# EUROPEAN PATENT SPECIFICATION

(11) **EP 2 155 223 B1**
(45) Date of publication and mention of the grant of the patent: **12.09.2012**
(21) Application number: 08751354.5
(22) Date of filing: 14.05.2008
(51) Int. Cl.: A61K 36/00, C11B 9/00

(54) **FORMULATION FOR FEVER REDUCING TREATMENT AND A METHOD AND KIT FOR USING THEREOF**
FORMULIERUNG FÜR EINE FIEBERSENKENDE BEHANDLUNG UND VERFAHREN UND KIT ZU IHRER ANWENDUNG
FORMULATION POUR TRAITEMENT RÉDUISANT LA FIÈVRE ET PROCÉDÉ ET TROUSSE L'UTILISANT

(30) Priority: 21.05.2007 US 939098 P
(43) Date of publication of application: 24.02.2010
(73) Proprietor: Microdel Idea Center Ltd., 52573 Ramat Gan (IL)
(72) Inventor: EZYON, Mazal, 78374 Ashkelon (IL)
(74) Representative: Modiano, Micaela Nadia
(86) International application number: PCT/IL2008/000666
(87) International publication number: WO 2008/142674

(56) References cited:
- US-A1- 2002 106 388
- US-A1- 2006 127 513
- AMABEOKU G ET AL: "Analgesic and antipyretic effects of Dodonaea angustifolia and Salvia africana-lutea" JOURNAL OF ETHNOPHARMACOLOGY, vol. 75, no. 2-3, 1 May 2001 (2001-05-01), pages 117-124, XP027380316 ELSEVIER SCIENTIFIC PUBLISHERS LTD, IE ISSN: 0378-8741 [retrieved on 2001-05-01]
- WIART C ET AL: "Antimicrobial activity of Acalypha siamensis Oliv. ex Gage" JOURNAL OF ETHNOPHARMACOLOGY, vol. 95, no. 2-3, 1 December 2004 (2004-12-01), pages 285-286, XP004990579 ELSEVIER SCIENTIFIC PUBLISHERS LTD, IE ISSN: 0378-8741 DOI: 10.1016/J.JEP.2004.07.014
- GARCIA M D ET AL: "Analgesic, antipyretic and antinflammatory effects of Achillea ageratum" PHYTOTHERAPY RESEARCH, vol. 11, no. 5, 1 August 1997 (1997-08-01), pages 376-379, XP002617956
- Diane: "Fever Home Remedies"[Online] 8 May 2005 (2005-05-08), XP002617957 Students projects Retrieved from the Internet: URL:http://www.susangaer.com/studentprojec ts/fever2.htm> [retrieved on 2011-01-21]
- UMMSAMIYAH.: 'Natural Fever Reducer.' MOTHERINGDOTCOMMUNITY FORUMS, [Online] vol. 4, 05 July 2006, page 46, XP008131930 Retrieved from the Internet: <URL:http://www.mothering.com/discussions/a rchive/index.php/t-479350.html> [retrieved on 2009-03-15]

## Description

### FIELD OF THE INVENTION

The present invention generally relates to the field of medical treatments. More particularly, the present invention relates to a new composition for use in fever reducing treatment by external means.

### BACKGROUND OF THE INVENTION

Fever, as commonly defined is body temperature higher than normal. Fever occurs when the hypothalamus, which is the part of the brain that controls and regulates the temperature of the body, raises the temperature over the normal level of about 37 degrees Celsius. The hypothalamus, while serving as the body's thermostat, sets the body temperature to a higher lever when an infection or another ailment is detected.

The white cells contained in the blood produce a substance called interleukin-1 when they digest microorganisms that may cause illness. Interleukin-1 impels the formation of prostaglandins that are the substances that directly trigger the hypothalamus to raise the body temperature and form fever. Thus, fever is one of the body's procedures for fighting pathogens. Consequently, there is usually no real reason to treat fever under 38 degrees Celsius.

It is also known that prior art fever reducing treatments, also acknowledged as antipyretic treatments of the Conventional Medicine, are based on oral application of medicines that belong to one of the four basic categories of medications: Aspirin, Ibuprofen, Acetaminophen, and Naproxen. These prior art antipyretic drugs additionally have a broad systemic activity therefore they are also used to relieve pains (analgesic), heal inflammations and cure swellings.

All these four basic categories of medications, which are classified as nonsteroidal anti-inflammatory drugs (NSAID), reduce fever by blocking the activity of the cyclooxygenase (COX) enzyme, thus inhibiting the synthesis of prostaglandin E2 in the central nervous system, consequently blocking peripheral pain impulses. It should be emphasized that medicines that belong to these categories are effective for only minor pain, inflammations and fever.

### SUMMARY OF THE INVENTION

The present invention is directed to an absorbing material as defined in claim 1, to a fever reducing formulation for use in application to the skin as defined in claim 4, and to the use of certain ingredients for the manufacture of a mixed formulation for reducing fever, as defined in claim 6.
More in particular, the present invention is directed to an absorbing material selected from the group consisting of a patch, a pad, a plaster, a towel, cloth, paper, a non-woven material, or a combination thereof for reducing fever, wherein said absorbing material is moistened with a fever reducing solution and with at least one fragrant masking ingredient, wherein said fever reducing solution comprises an aqueous solution of acetic acid at a concentration between 1% and 5%, and said fever reducing solution comprises an *Achillea spp.* sweating inducing extract.
More in particular, the present invention is also directed to a fever reducing formulation for use in application to the skin comprising:
an aqueous solution comprising acetic acid at a concentration between 1% and 5%,
at least one fragrant masking ingredient,
at least one emulgator,
an *Achillea spp.* sweating inducing extract,
wherein said solution and said plant extract are arranged to reduce fever while said at least one masking ingredient and said at least one emulgator are arranged to mask the smell of said acetic acid.
More in particular the present invention is also directed to the use of at least one fever reducing formulation comprising an aqueous solution comprising acetic acid at a concentration between 1% and 5%, an *Achillea spp.* sweat inducing extract, at least one fragrant masking formulation, and at least one emulgator for the manufacture of a mixed formulation for reducing fever by moistening an absorbing material with said mixed formulation and applying said absorbing material to a skin part of the body.

The present disclosure thus concerns a fever reducing formulation comprising an aqueous solution comprising acetic acid at a concentration between 1% and 5%, at least one fragrant masking ingredient, at least one emulgator, and at least one sweating inducing plant extract *of Achillea spp.* The solution and the plant extract are arranged to reduce fever while the at least one masking ingredient and at least one emulgator are arranged to mask the smell of the acetic acid. The present disclosure further discloses a method of reducing fever comprising the steps of mixing at least one fever reducing formulation with at least one fragrant masking formulation, moistening an absorbing material with the mixed formulation, and applying the absorbing material on a part of the body. The present disclosure further discloses a kit for reducing fever comprising an aqueous solution comprising acetic acid at a concentration between 1% and 5%, at least one fragrant masking ingredient, at least one emulgator, at least one sweating inducing plant extract, a container for combining the aqueous solution of acetic acid, the at least one fragrant masking ingredient, the at least one emulgator and the at least one sweating inducing plant extract, as well as an absorbing material for applying the combination to the skin, and an information and instruction sheet.

In embodiments, the absorbing material consists of a patch, a pad, a plaster, a towel, cloth, paper, or a combination thereof. The at least one sweating inducing plant extract comprises an extract of *Achillea spp..* In embodiments, the absorbing material is moistened with a fever reducing formulation and with a fragrant masking ingredient and is enclosed in a sealed package in a ready-to-use form. Upon opening the package, the moistened absorbing material may be immediately applied to the body.

### BRIEF DESCRIPTION OF THE DRAWINGS

The subject matter regarded as the invention will become more clearly understood in light of the ensuing description of embodiments herein, given by way of example and for purposes of illustrative discussion of the present invention only, with reference to the accompanying drawings (Figures, or simply "FIGS."), wherein:

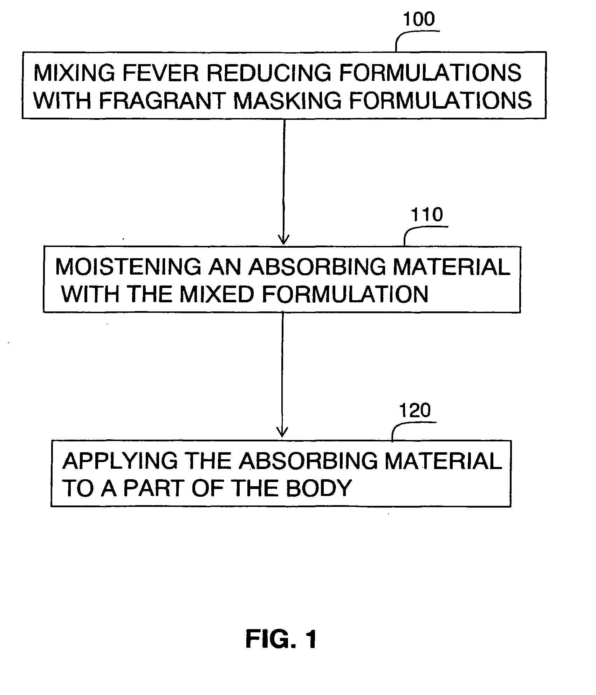

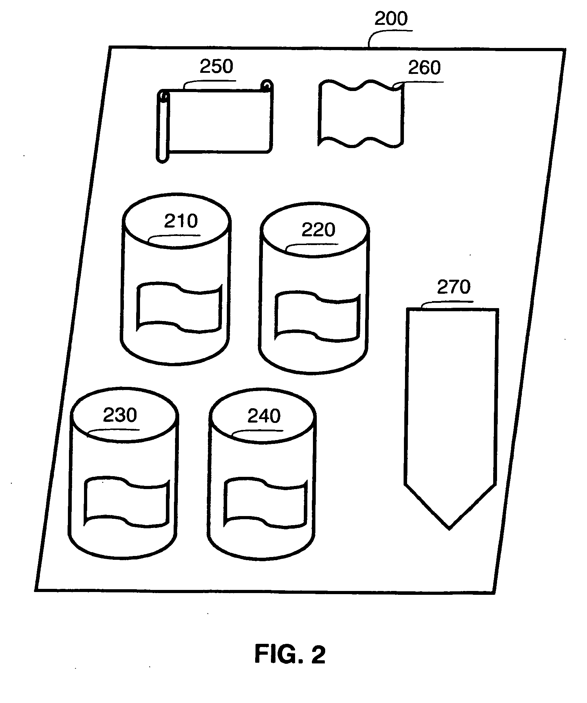

FIG. 1 is a flowchart illustrating a method for reducing fever, according to some embodiments of the-disclosure; and FIG. 2 is an illustration of a kit for reducing fever, according to some embodiments of the disclosure.

### DETAILED DESCRIPTIONS OF SOME EMBODIMENTS OF THE INVENTION

The present invention discloses a fever reducing formulation comprising an aqueous solution of acetic acid, an *Achillea spp.* sweating inducing extract and fragrant masking ingredients. The solution of acetic acid and the plant extract reduce fever while the fragrant masking ingredients mask the smell of the acetic acid. The formulation may be used to moisten an absorbing material which may be applied to the body.

Fig. 1 is a flowchart illustrating a method for reducing fever, according to some embodiments of the disclosure. The method comprises the steps:
- Mixing fever reducing formulations with fragrant masking formulations (step 100). Fever reducing formulations comprise an aqueous solution of acetic acid at a concentration between 1% and 5%, a sweating inducing *Achillea spp.* extract. Sweating may increase the heat dissipation from the body and thus reduce the patient's fever. Fragrant masking formulations may have a strong fragrance that mask the smell of the acetic acid solution, and may comprise pine oil, rose oil, lavender, jasmine, other essential oils.
- Moistening an absorbing material with the mixed formulation (step 110). The absorbing material is selected from the group consisting of a patch, a pad, a plaster, a towel, or made of cloth, paper or a non-woven material.
- Applying the absorbing material to a part of the body of the patient (step 120), possibly directly around the chest, back, arms or legs of the patient.

Fig. 2 is an illustration of a kit for reducing fever, according to some embodiments of the disclosure. The kit comprises an acetic acid vessel comprising an aqueous solution of acetic acid at a concentration between 1% and 5% 210, at least one fragrant vessel comprising at least one fragrant masking ingredient 220, at least one emulgator vessel comprising at least one emulgator, at least one extract vessel comprising at least one sweating inducing *Achillea spp.* extract. The kit further comprises a container 270 for combining and mixing the aqueous solution of acetic acid, fragrant masking ingredients, emulgators and sweating inducing *Achillea spp.* extract. The kit further comprises an absorbing material 250 and an information and instruction sheet. The kit is contained in a package 200. The solution of acetic acid and *Achillea spp.* extract reduce fever while the fragrant masking ingredients mask the smell of the acetic acid. At least one emulgator is chosen and configures to form an emulgation from the solution of acetic acid, fragrant masking ingredient and plant extract.

According to some embodiments of the invention, the absorbing material 250 is a patch, a pad, a plaster, a towel, or made of cloth, paper or a non-woven material. The sweating inducing plant extract comprises an extract of *Achillea spp..* The fragrant masking formulations may have a strong fragrance that mask the smell of the acetic acid solution, and may comprise pine oil, rose oil, lavender, jasmine, other essential oils.

According to some embodiments of the invention, the absorbing material 250 may be moistened with an aqueous solution of acetic acid at a concentration between 1% and 5%, at least one sweating inducing *Achillea spp.* extract, a fragrant masking ingredient and an emulgator, and packed within a sealed package in a ready-to-use form. Opening the package, the moistened absorbing material may be immediately applied to the body. According to some embodiments of the invention, the fragrant masking ingredient may be Johnson & Johnson 370, and the emulgator may be Dermaphyl Solplus.

According to some embodiments of the invention, the disclosed invention avoids undesirable side effects of prior art oral application medicines, such as nausea, and hemorrhage and diarrhea in the digestive system. A single high dose of Acetaminophen, for instance, may cause stomach and kidney problems, and, as a result be fatal. This may occur since both of these organs require COX for their normal functioning. It should be stressed that these medicines are especially dangerous for small children. Acetaminophen Ibuprofen and Aspirin, for example, are not given to children under 6 months, and can be dosed at time intervals of at least 6 hours, since they may develop health problems including Reyes syndrome that causes serious damage to the brain and the liver. Additionally, chronic use of the common fever and pain relief drugs can result in liver and kidney failure. Aspirin may also trigger an asthma attack in people with asthma.

Skin plays an important role in the dissipation of heat from the body, and is thus taking part in the mechanism of fever control determined by the hypothalamus. Body temperature is increased by prevention of heat loss due to vasoconstriction (narrowing of blood vessels) and reduced sweat. According to some embodiments of the disclosure, the disclosed invention for use in the reduction of fever avoids the side effects of the oral application medicines by applying a formulation to the skin by an external mean such as a patch, pad, plaster and towel. These are particularly significant for use in the treatment of children, where the antipyretic activity is a prominent objective of medicinal use of the above medicines.

Methods of treatment that act directly on the skin have the advantage of acting directly on one of the organs implicated in the production and dissipation of fever without affecting internal organs such as the liver and the stomach. One of these common folk methods is the use of a cloth soaked with low concentrated acetic acid, or vinegar, applied directly around the chest, back, arms or legs of the patient.

However, to many people, the smell of vinegar or acetic acid is considered to be unpleasant, therefore it is desirable to add at least one masking ingredients into the vinegar or acetic acid. Masking ingredients are substances that have a very strong fragrance that covers the original smell. These substances may be substances generally used in the cosmetic and body care industries such as pine oil, rose oil, lavender, jasmine, etc.

Breaking of fever is associated with vasodilatation (widening of blood vessels) and sweating. According to the invention, sweating inducing *Achillea spp.* extract is added to enhance fever reduction.

In the above description, an embodiment is an example or implementation of the inventions. The various appearances of "one embodiment," "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

It is understood that the phraseology and terminology employed herein is not to be construed as limiting and are for descriptive purpose only.

The principles and uses of the teachings of the present invention may be better understood with reference to the accompanying description, figures and examples.

It is to be understood that the details set forth herein do not construe a limitation to an application of the invention.

Furthermore, it is to be understood that the invention can be carried out or practiced in various ways and that the invention can be implemented in embodiments other than the ones outlined in the description above.

It is to be understood that where the claims or specification refer to "a" or "an" element, such reference is not be construed that there is only one of that element.

It is to be understood that where the specification states that a component, feature, structure, or characteristic "may", "might", "can" or "could" be included, that particular component, feature, structure, or characteristic is not required to be included.

Where applicable, although state diagrams, flow diagrams or both may be used to describe embodiments, the invention is not limited to those diagrams or to the corresponding descriptions. For example, flow need not move through each illustrated box or state, or in exactly the same order as illustrated and described.

Methods of the present invention may be implemented by performing or completing manually, automatically, or a combination thereof, selected steps or tasks.

The term "method" may refer to manners, means, techniques and procedures for accomplishing a given task including, but not limited to, those manners, means, techniques and procedures either known to, or readily developed from known manners, means, techniques and procedures by practitioners of the art to which the invention belongs.

The descriptions, examples, methods and materials presented in the claims and the specification are not to be construed as limiting but rather as illustrative only.

Meanings of technical and scientific terms used herein are to be commonly understood as by one of ordinary skill in the art to which the invention belongs, unless otherwise defined.

The present invention can be implemented in the testing or practice with methods and materials equivalent or similar to those described herein.

While the invention has been described with respect to a limited number of embodiments, these should not be construed as limitations on the scope of the invention, but rather as exemplifications of some of the preferred embodiments. Those skilled in the art will envision other possible variations, modifications, and applications that are also within the scope of the invention. Accordingly, the scope of the invention should not be limited by what has thus far been described, but by the appended claims and their legal equivalents.

## Claims

1. An absorbing material selected from the group consisting of a patch, a pad, a plaster, a towel, cloth, paper, a non-woven material, or a combination thereof for reducing fever, wherein said absorbing material is moistened with a fever reducing solution and with at least one fragrant masking ingredient, wherein said fever reducing solution comprises an aqueous solution of acetic acid at a concentration between 1% and 5%, and said fever reducing solution comprises an *Achillea spp.* sweating inducing extract.

2. The absorbing material of claim 1, wherein said absorbing material moistened with a fever reducing solution and with at least one fragrant masking ingredient, is enclosed in a sealed package in a ready-to-use form, and wherein opening the package, said moistened absorbing material may be immediately applied to the body.

3. The absorbing material of claim 1, further comprising at least one emulgator mixed with at least one fragrant masking ingredient, wherein said fragrant masking ingredient comprises at least one or more of the following: pine oil, rose oil, lavender, jasmine or essential oils.

4. A fever reducing formulation for use in application to the skin comprising:
an aqueous solution comprising acetic acid at a concentration between 1% and 5%,
at least one fragrant masking ingredient,
at least one emulgator,
an *Achillea spp.* sweating inducing extract,
wherein said solution and said plant extract are arranged to reduce fever while said at least one masking ingredient and said at least one emulgator are arranged to mask the smell of said acetic acid.

5. The formulation of claim 4, wherein said at least one fragrant masking ingredient comprise at least one or more of the following: pine oil, rose oil, lavender, jasmine or essential oils.

6. Use of at least one fever reducing formulation comprising an aqueous solution comprising acetic acid at a concentration between 1% and 5%, an *Achillea spp.* sweat inducing extract, at least one fragrant masking formulation, and at least one emulgator for the manufacture of a mixed formulation for reducing fever by moistening an absorbing material with said mixed formulation and applying said absorbing material to a skin part of the body.

7. The use of claim 6, wherein said absorbing material is one of the following: a patch, a pad, a plaster, a towel, cloth, paper, a non-woven material, or a combination thereof.

## Patentansprüche

1. Ein absorbierendes Material, das aus der Gruppe gewählt ist, die besteht aus einem Pflaster, einem Bauch, einem Verband, einem Tuch, einem Gewebematerial, Papier, einem Vliesmaterial, oder einer Kombination aus diesen, zum Senken von Fieber, wobei das absorbierende Material mit einer fiebersenkenden Lösung und mit mindestens einem angenehm riechenden, maskierenden Bestandteil benetzt ist, wobei die fiebersenkende Lösung eine wässrige Lösung aus Essigsäure in einer Konzentration zwischen 1% und 5% aufweist, und wobei die fiebersenkende Lösung einen Schwitzen herbeiführenden Extrakt aus Arten von *Achillea* (Scharfgabe) aufweist.

2. Das absorbierende Material nach Anspruch 1, wobei das absorbierende Material, das mit einer fiebersenkenden Lösung und mit mindestens einem angenehm riechenden, maskierenden Bestandteil benetzt ist, in einer dicht verschlossenen Packung in gebrauchsfertiger Form eingeschlossen ist, und wobei nach Öffnen der Packung das benetzte absorbierende Material sofort auf den Körper aufgebracht werden kann.

3. Das absorbierende Material nach Anspruch 1, das weiter mindestens einen Emulgator aufweist, der mit dem mindestens einen angenehm riechenden, maskierenden Bestandteil vermischt ist, wobei der angenehm riechende, maskierende Bestandteil mindestens eines oder mehrere der folgenden beinhaltet: Pinienöl, Rosenöl, Lavendel, Jasmin oder ätherische Öle.

4. Eine fiebersenkende Rezeptur für eine Verwendung zum Aufbringen auf die Haut, aufweisend:
eine wässrige Lösung, welche Essigsäure in einer Konzentration zwischen 1% und 5% aufweist,
mindestens einen angenehm riechenden, maskierenden Bestandteil,
mindestens einen Emulgator,
einen schwitzen herbeiführenden Extrakt aus Arten von Achillea (Scharfgabe),
wobei die Lösung und der Pflanzenextrakt angerichtet sind, um Fieber zu senken, hingegen der mindestens eine maskierende Bestandteil und der mindestens eine Emulgator angerichtet sind, um den Geruch der Essigsäure zu maskieren.

5. Die Rezeptur nach Anspruch 4, wobei der mindestens eine angenehm riechende, maskierende Bestandteil mindestens einen oder mehrere von den folgenden beinhaltet: Pinienöl, Rosenöl, Lavendel, Jasmin oder ätherische Öle.

6. Anwendung mindestens einer fiebersenkenden Rezeptur, die eine wässrige Lösung enthält, die Essigsäure in einer Konzentration zwischen 1% und 5%, einen Schwitzen herbeiführenden Extrakt aus Arten von *Achillea* (Scharfgabe), mindestens eine angenehm riechende, maskierende Rezeptur und mindestens einen Emulgator aufweist, für die Herstellung einer gemischten Rezeptur zum Senken von Fieber durch Benetzen eines absorbierenden Materials mit der gemischten Rezeptur und Aufbringen des absorbierenden Materials auf einen Hautteil des Körpers.

7. Die Anwendung nach Anspruch 6, wobei das absorbierende Material eines der folgenden ist: ein Pflaster, ein Bausch, ein Verband, ein Tuch, ein Gewebematerial, Papier, ein VliesMaterial, oder eine Kombination aus diesen.

## Revendications

1. Matériel absorbant sélectionné parmi le groupe qui consiste en un patch, un tampon, un plâtre, une serviette, tissu, papier, un matériel non-tissé, ou une de leur combinaison pour réduire la fièvre, dans lequel ledit matériel absorbant est humidifié par une solution réduisant la fièvre et par au moins un ingrédient de masquage parfumé, dans lequel ladite solution réduisant la fièvre comprend une solution aqueuse d'acide acétique à une concentration entre 1% et 5%, et ladite solution réduisant la fièvre comprend un extrait de *Achillea spp.* induisant la transpiration.

2. Matériel absorbant selon la revendication 1, dans lequel ledit matériel absorbant humidifié par une solution réduisant la fièvre et par au moins un ingrédient de masquage parfumé, est inclus dans un emballage scellé prêt à l'usage, et dans lequel après avoir ouvert l'emballage, ledit matériel absorbant humidifié peut être immédiatement appliqué sur le corps.

3. Matériel absorbant selon la revendication 1, comprenant en outre au moins un émulsifiant mélangé à au moins un ingrédient de masquage parfumé, dans lequel ledit ingrédient de masquage parfumé comprend au moins un ou plusieurs des ingrédients suivants : huile de pin, huile de rose, lavande, jasmin ou huiles essentielles.

4. Formulation réduisant la fièvre pour l'emploi dans l'application sur la peau comprenant :
une solution aqueuse comprenant de l'acide acétique à une concentration entre 1% et 5%,
au moins un ingrédient de masquage parfumé,
au moins un émulsifiant,
un extrait de *Achillea spp.* induisant la transpiration,
dans laquelle ladite solution et ledit extrait de plantes sont prévus pour réduire la fièvre alors que ledit au moins un ingrédient de masquage parfumé et ledit au moins un émulsifiant sont sont prévus pour masquer l'odeur dudit acide acétique.

5. Formulation selon la revendication 4, dans laquelle ledit au moins un ingrédient de masquage parfumé comprend au moins un ou plusieurs des suivants : huile de pin, huile de rose, lavande, jasmin ou huiles essentielles.

6. Emploi d'au moins une formulation réduisant la fièvre comprenant une solution aqueuse comprenant de l'acide acétique à une concentration entre 1% et 5%, un extrait de *Achillea spp.* induisant la transpiration, au moins une formulation de masquage parfumé, et au moins un émulsifiant pour fabriquer une formulation mixte pour réduire la fièvre en humidifiant un matériel absorbant par ladite formulation mixte et appliquer ledit matériel absorbant sur une partie de la peau du corps.

7. Emploi selon la revendication 6, dans lequel ledit matériel absorbant est l'un des suivants : un patch, un tampon, un plâtre, une serviette, tissu, papier, un matériel non-tissé, ou une de leur combinaison.
